# EUROPÄISCHE PATENTSCHRIFT

(11) **EP 1 991 065 B1**
(45) Veröffentlichungstag und Bekanntmachung des Hinweises auf die Patenterteilung: **25.06.2014**
(21) Anmeldenummer: 07711750.5
(22) Anmeldetag: 01.03.2007
(51) Int. Cl.: A23K 1/16, A23K 1/18

(54) **FERTIGFUTTER FÜR HAUSTIERE**
READY-TO-EAT FEED FOR DOMESTIC PETS
ALIMENT MIXTE POUR ANIMAUX DOMESTIQUES

(30) Priorität: 01.03.2006 DE 102006009373
(43) Veröffentlichungstag der Anmeldung: 19.11.2008
(73) Patentinhaber: AlzChem AG, 83308 Trostberg (DE)
(72) Erfinder: GASTNER, Thomas, 84518 Garching an der Alz (DE); KRIMMER, Hans-Peter, 84558 Kirchweidach (DE)
(74) Vertreter: Dey, Michael
(86) Internationale Anmeldenummer: PCT/EP2007/001783
(87) Internationale Veröffentlichungsnummer: WO 2007/098952

(56) Entgegenhaltungen:
- WO-A-91/07954
- WO-A-2005/120246
- WO-A-2006/092298
- US-A1- 2005 192 183

## Beschreibung

Gegenstand der vorliegenden Erfindung ist ein Fertigfutter für Haustiere, welches als ernährungsphysiologisch wirksamen Bestandteil eine Guanidinoessigsäure-Komponente enthält.

Guanidinoessigsäure (GAA) ist eine bei Tieren und auch im Menschen vorkommende körpereigene Substanz, welche bei der Biosynthese des Kreatins eine zentrale Rolle einnimmt. Kreatin kann sowohl durch die Nahrung aufgenommen als auch endogen gebildet werden. Die Biosynthese geht von Glycin und L-Arginin aus. Bei Säugetieren wird vor allem in den Nieren, aber auch in der Leber und der Bauchspeicheldrüse, durch das Enzym Aminotransferase die Guanidino-Gruppe des L-Arginins gespalten und eine N-C-N-Gruppe auf das Glycin übertragen. Das L-Arginin wird hierbei in L-Ornithin umgewandelt. Die so gebildete Guanidinoessigsäure wird im nächsten Schritt, bei Vertebraten geschieht dies ausschließlich in der Leber, mit Hilfe des Enzyms Transmethylase in Kreatin umgewandelt. Hierbei dient das S-Adenosylmethionin als Methylgruppen-Donor. Das Kreatin wird anschließend über den Blutkreislauf zu den Zielorganen transportiert. Der Transport durch die Zellmembran in die Zellen geschieht hierbei durch einen spezifischen Kreatin-Transporter.

Mehrere Arbeitsgruppen konnten bereits in den Fünfzigerjahren des letzten Jahrhunderts in klinischen Studien zeigen, dass die Verabreichung von Guanidinoessigsäure in Kombination mit Betain bei Herzkrankheiten einen positiven Einfluss auf den Krankheitsverlauf hat. Die Patienten berichteten von einer deutlichen Verbesserung ihres Allgemeinbefindens. Weiterhin wurde eine verbesserte Ausdauer bei körperlicher Belastung und eine erhöhte Muskelkraft schon nach kurzer Behandlungsdauer festgestellt. Auch berichteten die Patienten von einer verbesserten Libido. 200 Patienten wurde eine Dosis von 30 mg GAA/kg täglich über ein Jahr verabreicht. Nebenwirkungen konnten nicht beobachtet werden (Borsook H.; Borsook M.E.: The biochemical basis of betaine-glycocyamine therapy. In: Annals of western medicine and surgery 5(10), 825, 1951).

Die internationale Patentanmeldung WO 91/07954 A1 offenbart die Verwendung von Guanidinoessigsäure in Kombination mit Methionin oder S-Adenosylmethionin zur Erhöhung des Kreatinspiegels im Muskel. Als Einsatzbereich werden Zustände genannt, welche einen höheren Kreatinspiegel im Muskel erfordern. Es werden hierbei sowohl medizinische Anwendungen als auch der Bereich der Sporternährung beansprucht.

Hierbei wird die Behauptung aufgestellt, dass die Verabreichung von Kreatin keine Erhöhung des Kreatinspiegels bewirkt. Diese Behauptung konnte inzwischen durch zahlreiche Arbeiten widerlegt werden (z.B. Persky, A. M., Brazeau, G. A.: Clinical Pharmacology of the Dietary Supplement Creatine Monohydrate. In: Pharmacol Rev, 2001, 53, 161-176). Ein direkter Vergleich der Wirksamkeit von Kreatin und Guanidinoessigsäure wird in WO 91/07954 nicht offenbart.

Von Guanidinoessigsäure ist weiterhin bekannt, dass sie eine antibakterielle Wirkung besitzt und in Tierversuchen erfolgreich gegen bakterielle Infektionen (Staphyllococcus aureus) eingesetzt werden konnte ("Preparation for protecting mammals against infection"; Stanley Drug Products Inc., USA; Neth. Appl. (1976), 7 pp. NL 7411216).

Im Zusammenhang mit der Überdosierung von Methionin ist ebenfalls bekannt, dass damit verbundene negative Effekte durch die Gabe von Guanidinoessigsäure abgemildert werden können ("Interrelations of choline and methionine in growth and the action of betaine in replacing them". McKittrick, D. S., Univ. of California, Berkeley, Archives of Biochemistry (1947), 15, 133-55).

Die internationale Patentanmeldung WO 2004/000297 A1 beschreibt eine Mischung für die Ernährung oder für pharmazeutische Zwecke bei Säugern. Diese Mischung besteht aus einer Proteinfraktion, welche L-Serin enthält und als weitere Komponente Guanidinoessigsäure. Die Mischung soll hierbei frei von Glycin sein oder nach der Hydrolyse der Mischung ein Verhältnis von L-Serin zu Glycin von größer als 2,7:1 enthalten. Als mögliche Produktform werden Lösungen, Emulsionen, Suspensionen, Gele, Riegel, Süßigkeiten und vorzugsweise Pulver genannt. Es findet sich kein Hinweis auf die Verwendung von Guanidinoessigsäure als Fertigfutter für Haustiere.

Ein Verhältnis von L-Serin zu Glycin von größer 2,7:1 ist in einer kommerziell erhältlichen Tiernahrung für Haustiere nicht anzutreffen. Tierische Rohstoffe, wie z.B. Tiermehl, enthalten deutlich mehr Glycin als Serin ("Amino acids of meals of animal origin"; de Vuyst, A. Univ. Louvain, Belgum, Agricultura (Heverlee, Belgium) (1964), 12(1), 141-51). In pflanzlichen Rohstoffen ist das Verhältnis zwischen Glycin und Serin vorwiegend ausgeglichen.

Kreatin nimmt im Energiestoffwechsel der Zelle eine wichtige Rolle ein, wobei es als energiereiches Phosphokreatin neben dem Adenosintriphosphat (ATP) eine wesentliche Energiereserve des Muskels darstellt. Im Ruhezustand des Muskels kann ATP auf Kreatin eine Phosphat-Gruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion durch das Enzym Kreatinkinase, eine Phosphatgruppe auf Adenosindiphosphat übertragen und somit ATP zurückbilden. Dies wird auch als Lohmann-Reaktion bezeichnet.

Kreatin ist seit langem als geeignetes Nahungsergänzungs- und Futtermittel bekannt. Bei starker und über längere Zeit anhaltender Muskelarbeit sind die natürlich im Körper vorhandenen Kreatinvorräte rasch erschöpft. Insbesondere bei Leistungssportlern haben sich gezielte Kreatingaben positiv auf die Ausdauer und Leistungsfähigkeit ausgewirkt, wobei unerwünschte Anreicherungsprozesse im Körper oder nachteilige Abbauprodukte unbekannt sind. Der Grund hierfür ist darin zu sehen, dass Kreatin bei einer übermäßigen Zufuhr vom Körper als Kreatin und Kreatinin ausgeschieden wird.

Weiterhin ist bekannt, dass eine Kreatin-Supplementierung zu einer Erhöhung der Körpermasse führt. Dies ist zu Anfang auf eine vermehrte Aufnahme von Wasser in den Muskel zurückzuführen. Langfristig gesehen führt das Kreatin aber indirekt durch vermehrte Proteinsynthese oder einen verminderten Proteinkatabolismus in den Myofibrillen zu einer Erhöhung der Muskelmasse (Int J Sports Med 21 (2000), 139-145). Als Ergebnis erhält man somit eine erhöhte fettfreie Körpermasse.

Neben dem Kreatin selbst, also dem Kreatin-Monohydrat, haben sich zwischenzeitlich aber auch zahlreiche Kreatinsalze, wie das Kreatinascorbat, -citrat, -pyruvat und andere, ebenfalls als geeignete Nahrungsergänzungsmittel erwiesen. Stellvertretend seien an dieser Stelle das europäische Patent EP 894 083 B1 und die deutsche Offenlegungsschrift DE 197 07 694 A1 genannt.

Die für den Menschen als positiv nachgewiesenen Wirkungen entfaltet Kreatin auch bei Tieren, weshalb seine Verwendung in diversen Futtermitteln ebenfalls hinlänglich vorbeschrieben ist. Bereits 1923 wurden von Benedict und Osterberg Studien an Hunden durchgeführt. Hierbei wurde beobachtet, dass oral appliziertes Kreatin in einer täglichen Dosis von ca. 40 mg/kg über mehrere Wochen zu einem deutlichen Gewichtszuwachs führt. Weiterhin wurde eine positive Stickstoffbilanz beobachtet (The Journal of Biological Chemistry No.1 (1923), 229-252).

GB 2 300 103 lehrt die Verwendung von Kreatin in Form eines Hundebiskuits, wofür das Kreatin Monohydrat gemeinsam mit Fleisch in einer extrudierten Masse angeboten wird.

Aus der internationalen Patentanmeldung WO 00/67 590 A1 ist die Verwendung von Kreatin oder von Kreatin-Salzen als Futterzusatz für Zucht- und Masttiere, als Ersatz für Fleischmehl, Fischmehl und/oder antimikrobielle Leistungsförderer, Wachstumshormone sowie Anabolika vorgeschrieben. In WO 2005/120246 ist Guanidinoessigsäure und/oder deren Salze als Futtermittelzusatz beschrieben.

Da Kreatin Monohydrat aufgrund seiner schlechten Löslichkeit aber nur unzureichend bioverfügbar ist, wird seine gemeinsame Verwendung mit weiteren physiologisch aktiven Verbindungen, vorzugsweise in Salzform, empfohlen. Die deutsche Offenlegungsschrift DE 198 36 450 A1 hat die Verwendung stabiler Brenztraubensäure-Salze und insbesondere des Kreatinpyruvats in Formulierungen zum Gegenstand, die für die Tieremährung geeignet sind.

Kreatin ist für carnivore und omnivore Wildtiere eine natürliche Komponente ihrer Ernährung. So fressen Wölfe mit einem Körpergewicht zwischen 15 und 60 kg im Durchschnitt pro Tag 100-130 g Fleisch pro Kilogramm Körpergewicht. Frisches Fleisch enthält zwischen 3 und 6 g (23-46 mmol) Kreatin pro Kilogramm. Ein Wolf von 35 kg nimmt somit etwa 3,5 bis 4,5 kg frisches Fleisch auf, welches zwischen 10,5 und 27 g Kreatin enthält. Domestizierte Hunde kommen hingegen bei einem Körpergewicht von 35 kg mit etwa 1,25 kg Fleisch aus. Wird es in frischer und roher Form aufgenommen, enthält es zwischen 3,75 und 7,5 g Kreatin (Research in Veterinary Science 62 (1997), 58-62).

Neben seinen unbestrittenen positiven physiologischen Eigenschaften besitzt Kreatin aber auch den Nachteil, dass es in wässrigen Lösungen und feuchten Formulierungen, insbesondere bei hohen Temperaturen, eine ausgeprägte Instabilität besitzt, wobei es sich in Kreatinin umwandelt. Kommerziell hergestelltes Tierfutter wird zur Haltbarmachung bei der Verarbeitung stark erhitzt. So werden z.B. bei der Herstellung von trockenen Hunde- und Katzen-Biskuits die Rohstoffe in Extrudern auf Temperaturen von bis zu 190 °C erhitzt. Feuchtigkeit, Druck und Hitze gelatinisieren die enthaltene Stärke und die erhaltene Masse wird anschließend in die gewünschte Form gebracht. Die hohen Temperaturen bei der Verarbeitung und die Lagerung unter feuchten Bedingungen, wie z.B. in Dosenfutter, welches etwa 75-85% Wasser enthält, führt dazu, dass die Hauptmenge des enthaltenen Kreatins in Kreatinin umgewandelt wird. Dies wurde von Harris auch an kommerziellem Dosenfutter und Trockenfutter für Hunde gezeigt. Die untersuchten acht Dosenfutter enthielten nur noch Spuren an Kreatin (0,36 bis 1,93 mmol/kg). Auch in den Trockenfuttern wurden in der Mehrzahl der Proben Werte von 0,7 mmol Kreatin pro Kilogramm gemessen (Research in Veterinary Science 62 (1997), 58-62). Hieraus wird offensichtlich, dass Hunde und Katzen, welche mit kommerziellem Tierfutter (0,36-4,25 mmol Kreatin pro Kilogramm Futtermittel) ernährt werden, deutlich weniger Kreatin über die Nahrung aufnehmen, als dies bei einer natürlichen Ernährung mit Frischfleisch (23-46 mmol Kreatin pro Kilogramm) der Fall wäre.

Diese Instabilität von Kreatin ist auch hinsichtlich der oralen Aufnahme von Bedeutung. Der pH-Wert des Magens von 1 bis 2 kann je nach Verweilzeit zu einem deutlichen Abbau des Kreatins zu Kreatinin führen. So konnte beim Menschen gezeigt werden, dass nach einer oralen Applikation von Kreatin nur etwa 15 bis 30 % von der Muskulatur resorbiert werden können (Greenhaff, P.L.: "Factors Modifying Creatine Accumulation in Human Skeletal Muscle". In: Creatine. From Basic Science to Clinical Applikation. Medical Science Symposia Series Volume 14, 2000, 75-82)

Aus den bezüglich Kreatin geschilderten Nachteilen des Standes der Technik hat sich für die vorliegende Erfindung die Aufgabe gestellt, Verbindungen für Fertigfutter zu finden, welche nach Möglichkeit eine geringe Instabilität bei industriellen Verarbeitungsprozessen besitzen. Sie sollten nicht nur hohe Verarbeitungstemperaturen unbeschadet überstehen, sondern auch z.B. in Dosenfutter unter feuchten Bedingungen lagerstabil sein. Weiterhin sollte die Verbindung im Gegensatz zu Kreatin das saure Milieu des Magens unbeschadet überstehen und erst nach der Aufnahme in den Körper in Kreatin umgewandelt werden. Die eingesetzten Futterzusatzmittel sollten selbst keine physiologisch nachteiligen Wirkungen entfalten und leicht nachweisbar sein. Unter wirtschaftlichen Gesichtspunkten stand für die erfindungsgemäß zu verwendenden Substanzen mit im Vordergrund, diese auf wirtschaftlich günstige Weise herzustellen.

Gelöst wurde diese Aufgabe durch Fertigfutter für Haustiere, die als ernährungsphysiologisch wirksamen Bestandteil Guanidinoessigsäure und/oder Guanidinoessigsäure-Salze enthalten.

Überraschend wurde beim Fertigfutter festgestellt, dass die Guanidinoessigsäure-Komponenten tatsächlich das gemäß Aufgabenstellung an sie gerichtete Anforderungsprofil erfüllen, da sie in einfacher und wirtschaftlicher Weise herzustellen sind; im Gegensatz zu Kreatin bzw. Kreatin-Monohydrat weisen Guanidinoessigsäure und deren Salze auch in saurer Lösung, wie sie im Magen auftritt, eine deutlich höhere Stabilität auf und sie werden erst unter physiologischen Bedingungen in Kreatin umgewandelt. Überraschenderweise hat sich als besonders vorteilhaft herausgestellt, dass die im vorliegenden Zusammenhang beschriebene Guanidinoessigsäure und deren Salze im Gegensatz zum Kreatin somit tatsächlich erst nach der Resorption, vor allem in der Leber, in Kreatin umgewandelt werden. Somit wird der überwiegende Teil der eingesetzten Verbindungen, im Gegensatz zum bekannten Kreatin, nicht bereits im Vorfeld durch Instabilitätsreaktionen abgebaut und ausgeschieden, sondern tatsächlich den physiologischen Anwendungsbereichen zur Verfügung gestellt. Die Guanidinoessigsäure und deren Salze können gemäß Erfindung somit wiederum im Gegensatz zum Kreatin und dessen Derivaten bei identischer Wirkung mit deutlich geringerer Dosierung eingesetzt werden.

Weiterhin konnte gezeigt werden, dass die Guanidinoessigsäure unter Bedingungen, wie sie bei der industriellen Herstellung von Futtermitteln auftreten, eine sehr hohe Stabilität besitzt. Guanidinoessigsäure zeigt hierbei klare Vorteile gegenüber Kreatin. Weiterhin konnte gezeigt werden, dass Guanidinoessigsäure eine deutlich bessere Lagerstabilität als Kreatin besitzt. Diese Vorteile waren in ihrer Gesamtheit so nicht vorherzusehen.

Aufgrund der überraschend günstigen Eigenschaften der Guanidinoessigsäure-Komponente im beanspruchten Fertigfutter ist dieses nicht auf spezifische Darreichungsformen beschränkt. Vielmehr kommen Varianten in Form von Halbfeucht- und Nassfutter genauso in Frage, wie insbesondere Dosenfutter, was von der vorliegende Erfindung ebenfalls berücksichtigt wird.

Das Fertigfutter basiert vorzugsweise auf tierischen oder/und pflanzlichen Rohstoffen. Ferner enthält das Fertigfutter vorzugsweise Glycin. Besonders bevorzugt enthält das Fertigfutter nach der Hydrolyse Glycin in einem Verhältnis zu L-Serin von mehr als 1:2,7, vorzugsweise von 1:1 oder größer.

Wie bereits angesprochen, ist das erfindungsgemäße Fertigfutter überraschend gut lagerstabil, obwohl es auch höhere Wassergehalte aufweist. Das vorgeschlagene Fertigfutter hat einen Wassergehalt im Bereich zwischen 20 und 80 Gew.-%.

Die erfindungswesentliche Guanidinoessigsäure-Komponente kann erfindungsgemäß nicht nur in freier Form, also tatsächlich als Guanidinoessigsäure vorliegen, sondern auch als Salz bzw. in Form einer Anlagerungs- oder Komplexverbindung. Selbstverständlich sind auch sämtliche Mischformen dieser Verbindungstypen möglich.

Für das erfindungsgemäße Fertigfutter haben sich Guanidinoessigsäure-Salze als günstig erwiesen, die mit Asparaginsäure, Ascorbinsäure, Brenztraubensäure, Bernsteinsäure, Fumarsäure, Gluconsäure, Oxalsäure, Pyroglutaminsäure, 3-Nicotinsäure, Milchsäure, Zitronensäure, Maleinsäure, Schwefelsäure, Ameisensäure, Salzsäure und Phosphorsäure erhalten werden, wobei Kalium-, Calcium- oder Natriumguanidinoacetat besonders geeignet sind. Es können natürlich auch Mischungen aus Guanidinoessigsäure mit einem oder mehreren der oben genannten Salze eingesetzt werden oder Mischungen, die aus den oben genannten Salzen bestehen.

Als weiterer Vorteil hat sich herausgestellt, dass die Guanidinoessigsäure und deren Salze in einem relativ breiten Mengenbereich in Fertigfutter eingesetzt werden können. Bezogen auf das gesamte Fertigfutter sollte dieses die Guanidinoessigsäure-Komponente vorzugsweise in Mengen von 0,01 bis 20 Gew.-%, insbesondere in Mengen von 0,1 bis 1,0 Gew.-% und besonders bevorzugt in einer Menge von 0,2 bis 0,5 Gew.-% enthalten.

Selbstverständlich kann das Fertigfutter neben der Guanidinoessigsäure-Komponente auch noch andere Inhaltsstoffe, wie beispielsweise ebenfalls ernährungsphysiologisch aktive Komponenten und/oder Formulierungshilfsmittel bzw. Füllstoffe enthalten.

Dabei kann es in Abhängigkeit vom jeweiligen konkreten Verwendungsfall durchaus empfehlenswert sein, als weitere physiologisch aktive Komponente Methylgruppendonoren, wie Cholin, Betain und/oder Methionin, zuzusetzen.

Insgesamt werden Guanidinoessigsäure und deren Salze durch die vorliegende Erfindung neuen Verwendungszwecken in der Ernährung, insbesondere von Fleischfressem wie sie Hunde und Katzen darstellen, zugeführt, wobei sie im Gegensatz zu den bislang bekannten Kreatin-Verbindungen deutliche und überraschende Vorteile aufweisen.

Die nachfolgenden Beispiele verdeutlichen die Breite der vorliegenden Erfindung.

### Beispiele

### Beispiel 1:

Eine Mischung, bestehend aus 5.000 mg Guanidinoessigsäure und 5.000 mg Betain, wurde bei der Herstellung in 1 kg eines handelsüblichen Softfutters für Hunde eingearbeitet. Die Menge an Guanidinoessigsäure im Endprodukt war 0,5 Gew.-%.

### Beispiel 2:

Eine Formulierung, bestehend aus 2.500 mg Guanidinoessigsäure und 5.000 mg Betain, wurde in 1 kg einer typischen Rezeptur für Dosenfutter für Hunde eingebracht. Die Menge an Guanidinoessigsäure im Endprodukt war 0,25 Gew.-%.

### Beispiel 3:

Eine Formulierung, bestehend aus 2.000 mg Guanidinoessigsäure-lactat, 750 mg Carnitintartrat, 100 mg Succrosestearat, 160 mg Talkum und 1.090 mg Fructose, wurde in 1 kg einer Basismasse für Hundebiskuits eingebracht. Die Menge an Guanidinoessigsäure im Endprodukt war 0,2 Gew.-%

### Beispiel 4:

Als Masterbatch wurde in 1 kg einer handelsüblichen Katzendosenfuttermischung folgende Formulierung homogen eingebracht: 1.000 mg Guanidinoessigsäure, 400 mg Methionin, 2000 mg Cholin, 40 mg Magnesiumstearat, 25 mg Carboxymethylcellulose und 135 mg Lactose. Die Menge an Guanidinoessigsäure im Endprodukt war 0,1 Gew.-%

### Beispiel 5: Stabilität

### 5.1

Die Stabilität von Kreatin und Guanidinoessigsäure wurde unter Bedingungen verglichen, welche bei der Herstellung von industriell hergestellten Fertigfutter auftreten. Hierzu wurde ein Modellsystem für das Extrudieren einer feuchten Futtermasse bei 160 °C verwendet. Guanidinoessigsäure und Kreatin wurden in Wasser gelöst (pH 7) und im Autoklaven für 30 Minuten auf 160 °C erhitzt. Anschließend wurde der Gehalt an Kreatin und Guanidinoessigsäure bestimmt. Die Geschwindigkeit der Cyclisierungsreaktion von Kreatin zu Kreatinin und von Guanidinoessigsäure zu Glycocyamidin ist ausschließlich vom pH-Wert und der Temperatur abhängig, jedoch völlig unabhängig von der Konzentration.

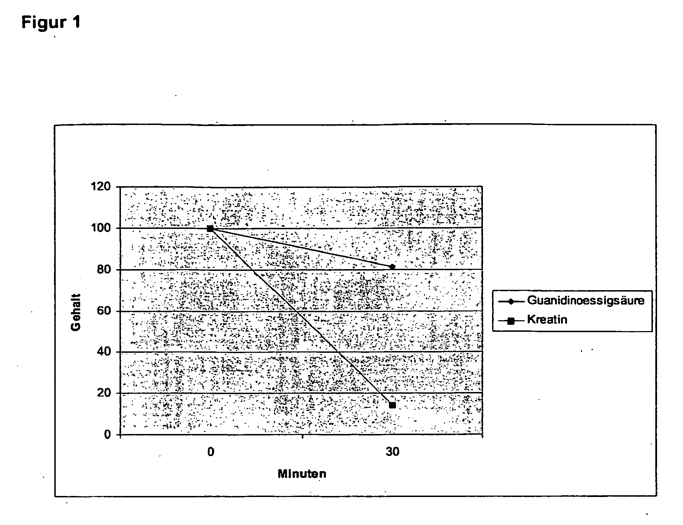

Das Ergebnis des Versuchs ist in Figur 1 gezeigt. Daraus ergibt sich, dass Guanidinoessigsäure eine signifikant höhere Stabilität als Kreatin bei den zur Herstellung von Tierfutter herrschenden Bedingungen aufweist. Während der Kreatingehalt nach 30 Minuten bei 160 °C weniger als 20 % des ursprünglichen Gehalts ist, liegen unter den gleichen Bedingungen noch mehr als 80 % Guanidinoessigsäure vor.

### 5.2

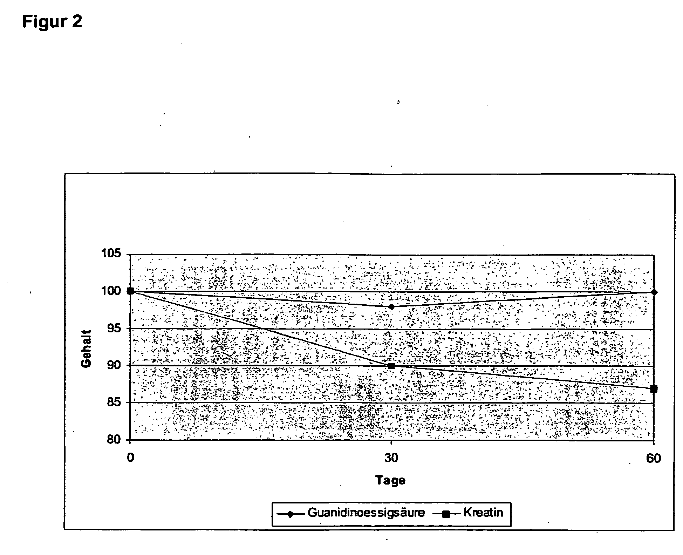

Die Stabilität von Kreatin und Guanidinoessigsäure wurde in Wasser bei pH 5 untersucht. Diese Bedingungen sind mit der Lagerung in Dosenfutter (75-85 % Wassergehalt) vergleichbar. Die Ergebnisse sind in Figur 2 gezeigt. Es ist zu erkennen, dass Guanidinoessigsäure eine deutlich bessere Lagerstabilität als Kreatin besitzt. Während bei Guanidinoessigsäure nach 60 Tagen kein Abbau zu beobachten ist, wird Kreatin nur noch zu 87 % wiedergefunden.

## Patentansprüche

1. Lagerstabiles Fertigfutter für Haustiere mit einem Wassergehalt zwischen 20 und 80 Gew.%, enthaltend mindestens eine Guanidinoessigsäure-Komponente als ernährungsphysiologisch wirksamen Bestandteil, erhältlich durch industrielle Verarbeitung eines Futtermittels, welches die Guanidinoessigsäure-Komponente enthält, wobei das Futtermittel, welches die Guanidinoessigsäure-Komponente enthält, zur Haltbarmachung bei der Verarbeitung hoch erhitzt wird.

2. Fertigfutter nach Anspruch 1,
**dadurch gekennzeichnet,**
**dass** es sich um Halbfeucht- und Nassfutter, insbesondere in Form von Dosenfutter handelt.

3. Fertigfutter nach einem der Ansprüche 1 oder 2,
**dadurch gekennzeichnet,**
**dass** es als Guanidinoessigsäure-Komponente Guanidinoessigsäure und/oder mindestens ein Salz, eine Anlagerungs- oder Komplexverbindung davon enthält.

4. Fertigfutter nach einem der Ansprüche 1 bis 3,
**dadurch gekennzeichnet,**
**dass** es sich bei der Guanidinoessigsäure-Komponente um Verbindungen zwischen Guanidinoessigsäure und Äpfelsäure, Asparaginsäure, Ascorbinsäure, Bernsteinsäure, Brenztraubensäure, Fumarsäure, Glukonsäure, α-Ketoglutarsäure, Oxalsäure, Pyroglutaminsäure, 3-Nicotinsäure, Milchsäure, Zitronensäure, Maleinsäure, Schwefelsäure, Essigsäure, Ameisensäure, 2-Hydroxybenzoesäure, L-Carnitin, Acetyl-L-Carnitin, Taurin, Betain, Cholin, Methionin und Liponsäure sowie Natrium, Kalium oder Calcium handelt.

5. Fertigfutter nach einem der Ansprüche 1 bis 4,
**dadurch gekennzeichnet,**
**dass** es die Guanidinoessigsäure-Komponente in gelöster Form enthält.

6. Fertigfutter nach einem der Ansprüche 1 bis 5,
**dadurch gekennzeichnet,**
**dass** es die Guanidinoessigsäure-Komponente in Mengen von 0,01 bis 20 Gew.-% und insbesondere in Mengen von 0,1 bis 1 Gew.-% und besonders bevorzugt 0,2 bis 0,5 Gew.-% enthält.

7. Fertigfutter nach einem der Ansprüche 1 bis 6,
**dadurch gekennzeichnet,**
**dass** es weiterhin einen Methyl-Gruppendonor, wie etwa Cholin und/oder Betain, enthält.

8. Fertigfutter nach einem der Ansprüche 1 bis 7,
**dadurch gekennzeichnet,**
**dass** es für Fleischfresser und insbesondere für Katzen und Hunde verwendet wird.

9. Verfahren zur Herstellung eines Fertigfutters für Haustiere nach einem der Ansprüche 1 bis 8, umfassend die industrielle Verarbeitung eines Futtermittels, welches die Guanidinoessigsäure-Komponente enthält, wobei das Futtermittel, welches die Guanidinoessigsäure-Komponente enthält, zur Haltbarmachung bei der Verarbeitung hoch erhitzt wird.

10. Lagerstabiles Dosenfutter für Haustiere mit einem Wassergehalt von 75 bis 85 Gew.-%, enthaltend mindestens eine Guanidinoessigsäure-Komponente als ernährungsphysiologisch wirksamen Bestandteil, erhältlich durch industrielle Verarbeitung eines Futtermittels, welches die Guanidinoessigsäure-Komponente enthält.

11. Verfahren zur Herstellung eines lagerstabilen Dosenfutters für Haustiere mit einem Wassergehalt von 75 bis 85 Gew.-%, enthaltend mindestens eine Guanidinoessigsäure-Komponente als ernährungsphysiologisch wirksamen Bestandteil, umfassend die industrielle Verarbeitung eines Futtermittels, welches die Guanidinoessigsäure-Komponente enthält.

## Claims

1. Storage-stable finished feed for domestic animals, having a water content of between 20 and 80 wt.% and containing at least one guanidinoacetic acid component as a nutritionally effective component obtained by industrially processing a feed containing the guanidinoacetic acid component, wherein the feed containing the guanidinoacetic acid component is heated to a high temperature during the processing in order to preserve it.

2. Finished feed according to claim 1, **characterised in that** the feed is semi-moist feed or wet feed, more particularly in the form of canned feed.

3. Finished feed according to either claim 1 or claim 2, **characterised in that** it contains guanidinoacetic acid and/or at least a salt, an addition compound or a complex compound thereof as the guanidinoacetic acid component.

4. Finished feed according to any of claims 1 to 3, **characterised in that** the guanidinoacetic acid component relates to compounds of guanidinoacetic acid and malic acid, aspartic acid, ascorbic acid, succinic acid, pyruvic acid, fumaric acid, gluconic acid, α-ketoglutaric acid, oxalic acid, pyroglutamic acid, 3-nicotinic acid, lactic acid, citric acid, maleic acid, sulphuric acid, acetic acid, formic acid, 2-hydroxybenzoic acid, L-carnitine, acetyl-L-carnitine, taurine, betaine, choline, methionine, lipoic acid, and sodium, potassium or calcium.

5. Finished feed according to any of claims 1 to 4, **characterised in that** it contains the guanidinoacetic acid component in a dissolved form.

6. Finished feed according to any of claims 1 to 5, **characterised in that** it contains the guanidinoacetic acid component in quantities of from 0.01 to 20 wt.% and more particularly in quantities of from 0.1 to 1 wt.% and particularly preferably 0.2 to 0.5 wt.%.

7. Finished feed according to any of claims 1 to 6, **characterised in that** it further contains a methyl group donor, for example choline and/or betaine.

8. Finished feed according to any of claims 1 to 7, **characterised in that** it is used for carnivores, and more particularly for cats and dogs.

9. Method for producing a finished feed for domestic animals according to any of claims 1 to 8, comprising industrially processing a feed containing the guanidinoacetic acid component, wherein the feed containing the guanidinoacetic acid component is heated to a high temperature during the processing in order to preserve it.

10. Storage-stable canned feed for domestic animals, having a water content of from 75 to 85 wt.% and containing at least one guanidinoacetic acid component as a nutritionally effective component obtained by industrially processing a feed containing the guanidinoacetic acid component.

11. Method for producing a storage-stable canned feed for domestic animals, having a water content of from 75 to 85 wt.%, containing at least one guanidinoacetic acid component as a nutritionally effective component, comprising industrially processing the feed containing the guanidinoacetic acid component.

## Revendications

1. Aliment préparé stable au stockage pour animaux domestiques, ayant une teneur en eau entre 20 et 80% en poids, contenant au moins un composant acide guanidinoacétique comme constituant actif au niveau de la physiologie nutritionnelle, obtenu par transformation industrielle d'un aliment pour animaux, qui contient le composant acide guanidinoacétique, où l'aliment pour animaux, qui contient le composant acide guanidinoacétique, est chauffé à une température élevée pour la conservation lors de la transformation.

2. Aliment préparé selon la revendication 1, **caractérisé en ce qu'**il s'agit d'aliments semi-humide et humide, en particulier sous forme d'aliment en boite.

3. Aliment préparé selon la revendication 1 ou 2, **caractérisé en ce qu'**il contient comme composant acide guanidinoacétique, l'acide guanidinoacétique et/ou au moins un sel, composé d'addition ou de complexation de celui-ci.

4. Aliment préparé selon l'une des revendications 1 à 3, **caractérisé en ce que** le composant acide guanidinoacétique consiste en des combinaisons entre l'acide guanidinoacétique et l'acide malique, l'acide aspartique, l'acide ascorbique, l'acide succinique, l'acide pyruvique, l'acide fumarique, l'acide gluconique, l'acide α-cétoglutarique, l'acide oxalique, l'acide pyroglutamique, l'acide 3-nicotinique, l'acide lactique, l'acide citrique, l'acide maléique, l'acide sulfurique, l'acide acétique, l'acide formique, l'acide 2-hydroxybenzoïque, la L-carnitine, l'acétyl-L-carnitine, la taurine, la bétaïne, la choline, la méthionine et l'acide lipoïque, ainsi que le sodium, le potassium ou le calcium.

5. Aliment préparé selon l'une des revendications 1 à 4, **caractérisé en ce qu'**il contient le composant acide guanidinoacétique sous forme dissoute.

6. Aliment préparé selon l'une des revendications 1 à 5, **caractérisé en ce qu'**il contient le composant acide guanidinoacétique en des quantités allant de 0,01 à 20% en poids et en particulier, en des quantités allant de 0,1 à 1% en poids, et de manière particulièrement préférée, de 0,2 à 0,5% en poids.

7. Aliment préparé selon l'une des revendications 1 à 6, **caractérisé en ce qu'**il contient en outre, un donneur de radical méthyle, comme la choline et/ou la bétaïne.

8. Aliment préparé selon l'une des revendications 1 à 7, **caractérisé en ce qu'**il est utilisé pour les carnivores, en particulier pour les chats et les chiens.

9. Procédé de préparation d'un aliment préparé pour animaux domestiques selon l'une des revendications 1 à 8, comprenant la transformation industrielle d'un aliment pour animaux, qui contient le composant acide guanidinoacétique, où l'aliment pour animaux, qui contient le composant acide guanidinoacétique, est chauffé à une température élevée pour la conservation lors de la transformation.

10. Aliment en boite stable au stockage pour animaux domestiques, ayant une teneur en eau allant de 75 à 85% en poids, contenant au moins un composant acide guanidinoacétique comme constituant actif au niveau de la physiologie nutritionnelle, obtenu par transformation industrielle d'un aliment pour animaux, qui contient le composant acide guanidinoacétique

11. Procédé de préparation d'un aliment en boite stable au stockage pour animaux domestiques, avec une teneur en eau allant de 75 à 85% en poids, contenant au moins un composant acide guanidinoacétique comme constituant actif au niveau de la physiologie nutritionnelle, comprenant la transformation. industrielle d'un aliment pour animaux, qui contient le composant acide guanidinoacétique
